# EUROPEAN PATENT APPLICATION

(11) **EP 2 071 024 A1**
(43) Date of publication of application: **17.06.2009**
(21) Application number: 07024324.1
(22) Date of filing: 14.12.2007
(51) Int. Cl.: C12N 9/16, C12N 7/00, C07K 1/14

(54) **Peptides of human prostatic acid phosphatase enhancing viral infectivity**

(71) Applicant: Pharis Biotec GmbH, 30625 Hannover (DE)
(72) Inventor: Forssmann, Wolf-Georg, 30175 Hannover (DE); Ständker, Ludger, 30625 Hannover (DE); Kirchhoff, Frank, 91054 Erlangen (DE); Münch, Jan, 89231 Neu-Ulm (DE)
(74) Representative: von Kreisler Selting Werner

(57) **Abstract**

Subject of the invention are peptides corresponding to a fragment of amino acids 85-120 of human prostatic acid phosphatase. The invention also relates to nucleic acids, antibodies, medicaments and diagnostics and their use and use of the peptides for the treatment and diagnosis of viral diseases, especially HIV disease.

## Description

### Background of the Invention

Viral diseases continue to be a major threat to public health. HIV/AIDS alone caused more than 2.8 million deaths in 2005 - more than self-inflicted injuries, violence and wars together. Hepatitis B and C viruses (HBV and HCV, respectively) have chronically infected more than 300 million individuals and cause hundred thousands of life threatening diseases each year. Measles and Dengue viruses kill about 600,000 individuals per annum (current WHO reports). New emerging diseases such as SARS, Ebola or the avian influenza, have far-reaching economic and public health consequences because they may cause novel pandemics. Other viruses, e.g. Lassa, Herpes 1 and 2, Cytomegalo, Parainfluenza or Yellow Fever Virus globally affect the quality of life of millions of individuals. Enormous financial efforts of governments, health organization and pharmaceutical industry to fight and prevent viral infections have led to the development of some effective vaccines and antiviral drugs in the last decades. However, the number of deaths caused by infectious diseases still increases. Licensed vaccines are only available for a very limited number of viral pathogens (e.g. HBV) and no effective vaccines against HIV or HCV - two of the most relevant global killers - will be available in the near future.
Various human pathogenic viruses are transmitted via sexual intercourse of an infected with an uninfected individual. Sexually transmitted viruses belong to the families of retroviridae [human immunodeficiency virus 1, HIV-2, xenotropic murine-like retrovirus (XMRV), hepadnaviridae (hepatitis B virus), flaviviridae (hepatitis C virus), papillomaviridae (human papilloma viruses) and herpesviridae (herpes simplex virus type 1 and 2 (HSV-1, HSV-2)]. The most common mode of HIV transmission is sexual contact between a male donor and a female recipient, with semen serving as the infectious fluid. Several strategies aiming to prevent virus transmission between individuals have been developed. Safer sex is a set of practices that are designed to reduce the risk of infection during sexual intercourse to avoid developing sexually transmitted diseases (STDs). Another strategy is to vaginally administrate compounds known as microbicides whose purpose is to reduce the infectivity of microbes, such as viruses or bacteria.
Viruses are obligate intracellular pathogens starting to replicate only after productive infection of living target cells. For some human pathogenic viruses e.g. HBV no reliable cell culture systems exist preventing the analysis of key aspects of the viral life cycle. Virus entry into the cell is mediated by viral proteins specifically binding cellular structures on the cell surface. These steps initiate conformational rearrangements in viral surface proteins resulting in the uptake of the virus into the cell.
Several diagnostically methods have been developed in the past decades to specifically detect virus. In rare conditions some viruses like HSV-1 can be isolated directly from specimens taken from a patient, most frequently blood or urine. However, in many cases isolation and hence characterization of various viruses is impossible due to the lack of efficient cell culture systems or too low viral infectivities.
Gene therapy approaches are often hampered by low transduction efficiencies of target cells, especially stem cells, by retroviral vectors. The transduction efficiency is crucial for efficient gene transfer into the target cell and is usually low when retroviral vectors are used for gene transfer in stem cells. Thus, there is also a need for an improved retroviral transduction system for successful delivery of the gene in appropriate cells.

### Summary of the Invention

The present invention is directed to methods and compositions for modulating infection of a cell by viruses and methods as well as compositions for treating a viral disease, for preventing virus transmission, to enhance infection rates of viruses in routine virological research, gene therapy approaches as well as virus isolation from patient specimens. The invention is based on the identification of a peptidic sequence termed PAP85-120 derived from human semen that forms amyloid fibrils drastically enhancing infection of enveloped viruses like HIV-1, HIV-2, HCV, XMRV and others. It is believed that aggregates of amyloidogenic peptide fibrils like SEVI (Muench et al, 2007.) or PAP85-120 in the semen may enhance infection of target cells by HIV and other sexually transmitted viruses like HCV, HBV or XMRV and hence contribute to the effective transmission of these viruses in the human population.

The present invention provides a method for modulating and enhancing viral infection rates of cells or tissues by administering PAP85-120 fibrils either directly to viral stock solutions or to cell culture. In the presence of PAP85-120 fibrils infection rates of HIV-1, HIV-2, MLV (mouse leukaemia virus), XMRV, HCV as well as certain retroviral particles carrying heterologous glycoproteins derived from lyssaviridae (vesicular stomatitis virus and rabies virus), filoviridae (ebola virus and marburg virus), retroviridae (HIV, MLV), coronaviridae (severe acute respiratory syndrome corona virus; SARS-CoV) are enhanced by 2 to 10,000 fold.

The present invention also provides a method to enhance transduction rates using viral vectors in in vitro and ex vivo gene therapy approaches.

*Other features and advantages of the invention will be apparent from the following detailed description, and from the claims.*

### Detailed Description of the Invention

Subject of the invention are peptides, nucleic acids, antibodies, medicaments and diagnostics and their use for the treatment and diagnosis of viral diseases, and the improvement of diagnostic methods, of virus isolation techniques and of transduction efficiencies with viral vectors in ex vivo and in vivo gene therapy.

The problem underlying the present invention is to provide improved or alternative medication, diagnostic tools and laboratory tools which overcome the above mentioned problems associated with viral diseases and gene therapy.
Surprisingly, the problem underlying the present invention is solved by peptides, nucleic acids, antibodies, medicaments, diagnostics and uses of any of claims 1-24.
The peptides of the invention are fragments of the amino acid residues 80-130 of human prostatic acid phosphatase (hPAP), more preferably of residues 85-120, which promote viral infection of a cell. The numbering refers to the protein sequence in its unprocessed form, including the pro-sequence. The human prostatic acid phosphatase (hPAP; Swissprot: locus PPAP_HUMAN, accession P15309) in its processed form consists of 354 amino acids with a calculated molecular mass of 41126 Da (Vihko et al.; 1988, Sharief & Li; 1992). The protein hPAP itself and the nucleic acid encoding it are not subject of the invention. The present invention is based on the discovery that hPAP fragment PAP85-120 present in semen can form amyloid like fibrillar structures that enhance the infectivity of cells by enveloped viruses like HIV-1, HIV-2, HBV, HCV as well as pseudotyped virions carrying glycoproteins of MLV, Ebola, SARS, Marburg, VSV or Rabies virus.
In general, peptides with partial sequences of the 51 residue section of amino acids 80-130 of the hPAP amino acid sequence are subject of the invention. Preferably, the peptides comprise at least 12, in other embodiments at least 20, 25, 30 or 50 amino acids. The peptides comprise no more than 50, preferably no more than 47 or 45 amino acids. Especially preferred is the peptide PAP85-120 of amino acid sequence SEQ ID No. 1. (Seq ID No. 1: PAP85-120 IRKRYRKFLNESYKHEQVYIRSTDVDRTLMSAMTNL). For example, the synthetic PAP85-120 peptide forms amyloid like fibrillary structures when dissolved in H20, medium or conventional buffers such as PBS either spontaneously or after agitation.

Further embodiments of the invention are peptides derived from PAP85-120 peptides with at least one of the following sequence variations:
- a deletion of amino acids at the N- and/or C-terminus, wherein the peptide consists of at least 25, preferably 31 to 35 amino acids.
- an addition of up to 10 amino acids, preferably 1, 2, 3 or 5 amino acids at the N- and/or C-terminus.
- an amino acid exchange of up to 5, preferably of 1, 2 or 3 amino acids; the amino acid exchange being preferably a conservative exchange.
- an amino acid insertion or deletion within the sequence of up to 3, preferably 2 or 1 amino acids.

In a conservative amino acid exchange, an amino acid is replaced by another amino acid with similar properties. Conservative amino acid exchanges are preferably within one of the groups of Amino acids with unpolar side chains: A, G, V, L, I, P, F, W, M Uncharged amino acids with polar side chains: S, T, G, C, Y, N, Q Amino acids with aromatic side chains: F, Y, W Positively charged amino acids: K, R, H Negatively charged amino acids: D, E.

Further embodiments of the invention are peptides derived from PAP85-120 or its derivatives as outlined above with at least one of the following covalent modifications:
- acylation, acetylation, linkage to a non-peptidic macromolecular carrier group; preferably at the N-teminus.
- amidation, linkage to a non-peptidic macromolecular carrier group; preferably at the C-terminus
- glycosylation; preferably at amino acid side chains.
- linkage to an adaptor protein, which promotes uptake of the peptide into cells or linkage to a hydrophobic group, preferably a lipid, a fatty acid, a dansyl, a carbobenzoxyl or a t-butyloxycarbonyl group.
- oxidation, sulphatization, esterification, lactone formation and/or phosphorylation.

Preferred macromolecular carrier groups are polyethylene glycol (PEG), polyoxyalkylene glycol, polysorbate esters, mannan, amylopectin, pullulan, hydrogelnanoparticles of self aggregated hydrophobized polysaccharides, polylysine or albumine.

Subject of the invention are also retro, inverso or retro-inverso peptides of PAP85-120 and peptides obtainable by multiple synthesis which have the biological activity of PAP85-120 peptides. The peptides may comprise at least one D-amino acid as well as iminoamino acids and rare amino acids, such as hydroxylysine, homoserine and ornithine. The invention also relates to peptide mimetics of the peptides according to the invention. These are characterized by a modification of one or more peptide bonds, for example, by a reverse peptide bond or by an ester bond.

The peptides of the invention promote viral infection of a cell. As used herein, "viruses" relates to natural occurring viruses and virus particles as well as artificial viruses and virus particles, which may for instance comprise a nucleic acid designed for gene therapy. In preferred embodiments, the viruses are retroviruses and retroviral particles such as oncoviruses, lentiviruses and foamy viruses. Generally, the peptides of the invention are especially useful to enhance infection of target cells with replication competent viruses. Specific viruses are HIV-1 (Human Immunodeficiency Virus Typ 1), HIV-2, Simian Immunodeficiency Viruses (SIV) as well as the oncovirus Murine Leukemia Virus (MuLV), xenotropic murine-like retrovirus (XMRV) and Foamy Virus. The peptides also promote the infection of HIV-variants, such as X4, R5 and dual-tropic variants, molecular HIV clones and various HIV-1 subtypes. The peptides also favour infection with Hepatitis B Virus (HBV) and Hepatitis C Virus (HCV) or retroviral particles that were pseudotyped with heterologous envelope proteins derived from Vesicular Stomatitis Virus (VSV), MuLV, Ebola, HIV-1, Rabies Virus, Marburg and MuLV. Therefore the peptides of the invention increase transduction efficiencies of retrovirus based gene therapy systems.

In other preferred embodiments of the invention, the peptides favour infections with flaviviruses such as yellow fever virus, Dengue virus, West-Nile virus, tick-borne encephalitis virus, hepatitis C and G virus, swine fever virus, with toga viruses such as rubella virus, with corona viruses such as SARS virus, with caliciviruses such as Norwalk virus and hepatitis E virus, with rhabdoviruses such as vesicular stomatitis, Indiana virus and rabies virus, with paramyxoviruses such as human parainfluenza virus I, mumps virus, measles virus and human respiratory syncytial virus, with filoviruses such as Marburg virus and Ebola virus, with orthomyxoviruses such as influenza A/B/C viruses, with arenaviruses such as Lassa virus, with herpesviruses such as herpes simplex viruses 1 and 2, varizella zoster virus, cytomegalovirus, Epstein-Barr virus, human herpesviruses 6, 7 and 8, with poxviruses such as vaccinia virus, with reoviruses such as rotaviruses, with picornaviruses such as poliovirus, rhinovirus, hepatitis A virus, encephalomyocarditis virus and foot-and-mouth disease virus, with papovaviruses such as polyomaviruses and pappilom virus, and with adenoviruses.

The activity of the peptides can be measured for instance in assay systems aimed to detect virus infection rates e.g. using chemiluminescence based assays or fluorescence techniques as well as direct detection of viral particles by ELISA. Preferably, the peptides, for instance at a concentration of 1 or 5 µg/ml, enhance viral infection into cells at least by a factor of 2, more preferred by a factor of 3, 5 or 100 when compared to viral infection into the cells in the absence of the peptide. When target cells are infected with a low multiplicity of infection, usually PAP85-120 enhances viral infection rates by several orders of magnitude. hPAP is secreted in high amounts into seminal fluid (Rönnberg et al.; 1981) and due to its pH optimum of 4-6, hPAP is categorized as an acid phosphatase. Enzyme concentrations are often elevated in patients with prostate carcinomas and are correlated with tumour progression (Choe et al.; 1980, Griffiths 1980, Vihko et al.; 1980, 1981, and 1985). hPAP was a main diagnostic marker for prostate cancer until the arrival of the prostate-specific antigen.

As a large quantity of hPAP is present in human semen, it has been suggested to have a physiological role in fertility (Coffey & Pienta 1987). The serum levels of hPAP are very low in healthy individuals (0-5 ng/ml) (Cusan et al.; 1994), but are often elevated in malignant and benign prostatic disease (Choe et al.; 1980, Griffiths 1980, Vihko et al.; 1980, 1981, and 1985). As such, immunoassays for hPAP serum levels are useful in monitoring disease progression in prostate cancer.
It is assumed that fibrillary PAP85-120 binds to viral or cellular membranes and activates processes that are essential for virus entry into the cell. The peptides, nucleic acids and antibodies of the invention are thus useful as therapeutics or diagnostics of viral infection, prostatic dysfunction, carcinoma or the susceptibility of a patient to a viral infection as well as to prevent virus transmission by sexual intercourse. The peptides, nucleic acids and antibodies of the invention are also useful to enhance infectivities of retroviral vectors that are used for gene delivery into cells or gene therapy approaches. They favour the infection of permissive target cells by retroviruses, e.g. HIV-1, HIV-2, SIV and other enveloped viruses like Ebola virus, Vesicular Stomatitis Virus (VSV), Mouse Leukemia Virus (MLV), and Hepatitis B Virus. The peptides of the invention exhibit dramatic enhancing effects on viral particle infectivity or fusion capability, such as the ability to favour HIV transmission to uninfected CD-4 + cells. Because of the high concentrations of hPAP and thus PAP85-120 in human sperm, PAP85-120 fibrils probably also facilitates HIV, HBV and HCV transmission from seropositive individuals to healthy sex partners as well. The peptides of the invention may be used as markers for the probability of viral transmission during sexual intercourse.

The peptides are thus used for a broad range of therapeutic and diagnostic applications and are valuable laboratory tools for the performance and study of entry of viruses into cells.

A preferred embodiment of the invention is the use of PAP85-120 as general enhancers of viral infection or transduction efficiencies for routine laboratory practice or gene therapeutic approaches based on viral vector systems. The peptides are enhancing the entry of retroviral vectors designed for gene therapy into cells *in vitro* or *in vivo.* They may be administered in combination with a vector for gene therapy and mediate entry of the vector into the target cell. The peptides are also useful *in vitro* because they accelerate the uptake of viruses into cells. They are thus useful as a tool for studying viruses and their mechanisms of action.

Another embodiment of the invention is the use of PAP85-120 for diagnostic approaches, especially those of viruses like HIV-1. The virus particles are interacting with the fibrillary PAP85-120. They can thus be used to isolate viral particles from samples like serum, blood, plasma, sperm or tissues derived from HIV infected humans or SIV infected primates. One important issue in HIV-1 diagnostic is the reisolation of viruses directly from blood or cell samples derived from HIV-1 infected individuals. In the presence of PAP85-120, successful virus isolation is favoured several times compared with routine diagnostic methods. Preferred methods are binding affinity assays and methods to remove viruses quantitatively from solutions suspected or known to comprise viruses in order to obtain safe solutions. In such methods, the peptides of the invention are preferably covalently bound to a support or a column.

The invention also relates to polynucleotides coding for the peptides according to the invention, such polynucleotides being preferably constituted of DNA, RNA, genomic DNA or PNA.

Another aspect of the invention relates to vectors containing the polynucleotide according to the invention, and genetically engineered host cells containing the vector according to the invention.

Recombinant or synthesized PAP85-120 peptides can be used to enhance in general the entry of retroviral particles (oncoviruses, lentiviruses and Foamy viruses) and Hepatitis B Virus into target cells. PAP85-120 can also be used as a general enhancer of the infection/transduction rate of retroviral core particles that carry foreign envelope glycoproteins (pseudoparticles) like the G protein of the Vesicular Stomatitis Virus (VSV-G), the Env protein of MuLV, HBV HBsAg (Hepatitis B Virus surface antigen), Ebola Virus spike protein or envelope proteins from different HIV-1, HIV-2 and SIV variants. PAP85-120 favours the infection rates of all analyzed retroviral particles, usually about 2 to 500 times. This allows to perform infection experiments, especially in primary cells, that have not been feasible before. The peptides of the invention are thus useful as laboratory tools *in vitro.*

Recombinant or synthesized PAP85-120 peptides can also be used for diagnostic approaches i.e. isolation of HIV-1 or SIV from infected individuals. PAP85-120 lowers the threshold for a detectable retroviral infection by at least 2 to 3 orders of magnitude. Thus, in the presence of PAP85-120, the sensitivity for a successful virus reisolation from patient samples is higher than without PAP85-120. PAP85-120 lowers the detection limit in HIV diagnostics for the detection of replication competent HIV-1 from infected individuals. For example PAP85-120 allows a more efficient reisolation of virus from PBMC of HIV-1 infected individuals by cocultivation with indicator cells compared to samples without PAP85-120. PAP85-120 also allows to efficiently reisolate virus from cell free samples of HIV-1 patients.

Recombinant or synthesized PAP85-120 peptides can also be used to enhance gene delivery rates in *ex vivo* or *in vivo* gene therapy approaches based on retroviral vector systems. The generation of highly infectious retroviral vectors for gene therapy, especially for ex vivo gene therapy of stem cells is a difficult procedure. The transduction efficiencies of retroviral vectors for stem cells are also low. In the presence of PAP85-120, however, stem cells and cell lines can be efficiently transduced with retroviral vectors, resulting in higher efficiencies for gene delivery into the target cell compared to samples containing no PAP85-120.

Expression vectors encoding PAP85-120 and/or derivatives thereof can be used to transiently or stably transfect cell lines to produce recombinant PAP85-120 peptides that can be purified from cell lysates or supernatants. Mono- or polyclonal antibodies specifically recognizing monomeric or fibrillary PAP85-120 and/or derivatives can be used to measure PAP85-120 concentrations in human sperm or seminal fluid as markers for prostatic cancer and the probability of HIV transmission. PAP85-120 specific antibodies can also be used as medical compounds to neutralize PAP85-120 activity in human sperm thus preventing HIV and HBV transmission by sexual intercourse.

The peptides according to the invention can be obtained by peptide synthesis, by a purification method from cells transfected with cDNA of peptide sequences or by a purification method starting from human sperm. In a preferred method, seminal fluid derived from sperm is subjected to cation exchange and reverse phase chromatography.

The peptides or antibodies according to the invention are preferably used in medicinal formulations. The medicinal formulation contains one or more of the peptides or antibodies according to the invention, or a physiologically acceptable salt of the peptides. Medicinal formulations can contain pharmaceutically usual auxiliary agents which contribute, for example, to the solubility, stability or sterility of the medicament or increase the efficiency of uptake into the body.

The form and composition of the medicament which contains the peptides or antibodies depends on the route of administration. Preferably, galenic formulations and application forms are selected in which the peptides or antibodies arrive at the target site in a non-degraded condition. The medicament can be administered locally as injection, drops, spray, tablets, suppositories, cream, ointments, gel etc. It is possible to perform the administration as a bolus or repeatedly over a period of time.

### Brief Description of the Drawings

Figure 1 is a drawing showing the isolation of PAP85-120 from a human semen derived peptide library.
Figure 2 shows the PAP85-120 sequence and PAP fragments obtained by mass spectrometric analysis.
Figure 3 is a diagram showing that PAP85-120 forms Thioflavin T binding fibrils when agitated. The freshly dissolved monomeric peptide does not contain detectable amounts of fibrils.
Figure 4 is a diagram showing that just the fibrillary but not a freshly dissolved PAP85-120 solution enhances HIV-1 infection.
Figure 5 shows that monomeric as well as fibrillary PAP85-120 do not exert cytotoxic effects.
Figure 6 is a diagram showing that fibrillary PAP85-120 dose dependently activates HIV infection independently of the coreceptor usage.
Figure 7 demonstrates that PAP85-120 fibrils enhance X4 and R5 tropic HIV-1 infection of PBMC and macrophages.
Figure 8 shows that PAP85-120 fibrils enhance HIV-1 infection independently of the HIV-1 subtype.
Figure 9 demonstrates that PAP85-120 fibrils lower the viral threshold required to allow productive HIV-1 infection.
Figure 10 shows that PAP85-120 fibrils favour MuHIV and HIV-2 infection as well.
Figure 11 demonstrates that PAP85-120 fibrils enhance infection of retroviral particles carrying the VSV-G or MLV glycoprotein.
Figure 12 shows that PAP85-120 fibrils enhance Hepatitis C Virus infection.
Figure 13 demonstrates that PAP85-120 fibrils favour XMRV infection.
Figure 14 shows that PAP85-120 fibrils enhance HIV attachment to cells.
Figure 15 demonstrates that only the precipitable fraction, but not the clear supernatant of an agitated PAP85-120 solution enhances HIV-1 infection.

### The invention is further described by means of the following

### examples.

### Examples

### Example 1 - Isolation of the HIV-1 infection promoting PAP85 peptide

### Purification from Human Sperm

For the generation of a peptide library derived from human whole sperm, 130 ml human sperm was collected from different healthy donors (n>10). For collection of sperm, healthy volunteers ejaculated whole sperm into a 50-100 ml plastic tube and the sperm was stored at -20 °C immediately after ejaculation. For preparation of proteins and peptides the defrozen sperm was mixed with 150 ml of ice cooled extraction buffer (1 M acetic acid, 2 M NaCl, 20 mM ascorbic acid, 0.1 mM EDTA) and homogenized (2 x 1 min at 13 500 and 20 500 upm) using an ultra turrax (IKA-Ultra-Turrax Typ 125) on ice. After addition of 60 ml extraction buffer and 20 ml water and pH adjustment to 3 using HCI the peptides/proteins were extracted by stirring for 3 h at 4°C. The homogenized extract was centrifuged at 16 000 g at 4°C over 40 min and the supernantant was used for further protein/peptide enrichment. The pellet was resuspended in extraction buffer, stirred for 2 additional h, centrifuged again and the obtained supernatant unified with the first supernatant. The unified supernatants were ultrafiltered/diafiltered using a polysulfon membrane (ultrasart SM 146501E-SG; Sartorius, Göttingen, Germany) with a molecular cut-off of 50 kDa to separate high molecular weight proteins (> 50 kDa) from the peptide fraction. The obtained peptide filtrate was conditioned for chromatography by dilution with water to a volume of 30 L and pH adjustment with HCI to 2.5. The conductivity was 4.8 mS/cm.

### Cation Exchange Chromatography:

For a first separation, a cation exchange column (Fractogel TSK SP 650 (S), Merck, Darmstadt, Germany; 20-50 microm particle size; 860 ml column volume) was equilibrated with water, pH 2.5 after conditioning with 0.5 M NaOH and 0.5 M HCI. The conditioned permeate was then loaded on the cation exchange column (flow rate: 25 ml/min). After washing with 33 L water, pH 2.5, the peptides/proteins were eluted using the following buffers:
1. 0.1 M citric acid, pH 3.6, volume: 2 L
2. 0.1 M acetic acid + 0.1 M sodium acetate, pH 4.5, volume: 1.9 L
3. 0.1 M malic acid, pH 5.0, volume: 2.3 L
4. 0.1 M malonic acid, pH 5.6, volume: 1.7 L
5. 0.1 M NaH₂P0₄, pH 6.6, volume: 2.5 L
6. 0.1 M Na₂HPO₄ pH 7.4, volume: 1.7 L
7. 1 M ammonium acetate, pH 7.0, volume: 1.6 L
8. water, pH 7.0, volume: 1.5 L
9. 0.1 M NaOH, pH 13, volume: 1.0 L

The obtained eluates were partially unified and further separated by reverse phase chromatography.

### First Reverse Phase Chromatography:

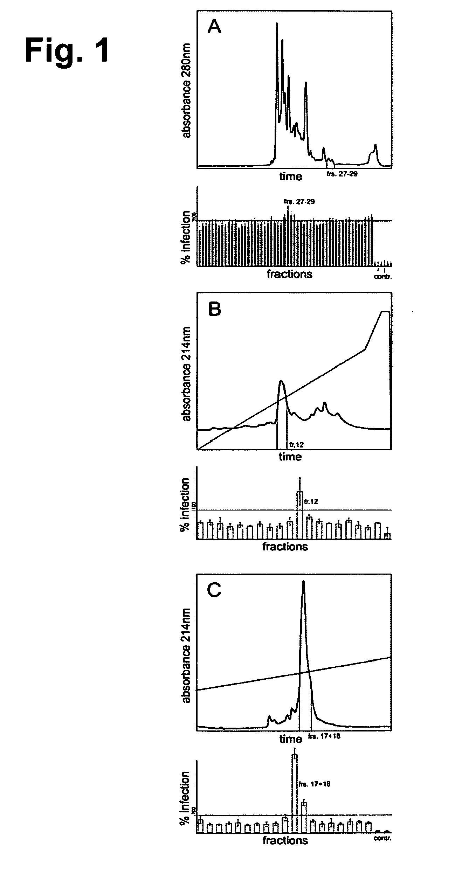

The second separation was carried out on a reverse phase (RP) chromatography column (C18, 47 x 300 mm i.d., 10-20 micron particle size, 30 nm pore size, Vydac, Hesperia, CA, USA) (exemplarily shown for eluate 7 in Fig. 1B). The eluates 1 to 9 were applied to the RP C18 column. The chromatography of the wash eluate was termed pool 0, the chromatography of the unified eluates 1 and 2 were termed pool 1, the chromatography of the unified eluates 3 and 4 were termed pool 2, chromatography of eluate 5 was pool 3, the chromatography of eluate 6 was termed pool 4 and the chromatography of the unified eluates 7-9 were termed pool 5. The separation was carried out in seven different runs and the peptides were eluated in gradient elution under identical conditions:
Flow rate: 40 ml/min
Buffer A: 10 mM HCI
Buffer B: A + 80% (v/v) acetonitrile
Gradient elution: from 100% A to 60 % B in 47.5 min
from 60% B to 100% B in 2.5 min

Fractions of 50 ml were collected and each run resulted in 40 peptide containing fractions. In total 240 fractions were obtained and from each fraction an aliquot corresponding to 1.3 ml equivalent of sperm (0.5 ml fraction volume) was taken and immediately lyophilized. The original fractions were immediately stored at -20°C prior to further chromatographic separation. Testing of the fractions:
The lyophilized peptide fractions from pool 4 containing 1.3 ml sperm equivalent were reconstituted in 33 µl of FBS (foetal bovine serum; Invitrogen; #10270-106) free D-MEM (Invitrogen, #41965-039) containing 100 units/ml penicillin G and 100 µg/ml Streptomycin sulphate (Pen/Strep) (Invitrogen, #15140-163). 4000 P4-CCR5 cells (Charneau et al. ; 1994.) were seeded in 96 well flat bottom plates (Greiner; #655180) in a total volume of 100 µl in DMEM (10% FBS, Pen/Strep). P4-CCR5 cells express HIV-1 receptors CD4, CCR5 and CXCR4 and stably contain a LTR-lacZ construct. After successful infection of these cells with HIV-1, the virally encoded Tat protein is expressed, which transactivates viral gene expression via binding to the LTR (Charneau et al. ; 1994). Thus Tat also activates the expression of the cell encoded LTR-IacZ, resulting in the production of the enzyme β-galactosidase in infected cells. The activity of the enzyme is directly proportional to viral infectivities. The next day, medium was removed, 30 µl of fresh medium and 10 µl of dissolved peptide fractions were added. After 4 h incubation at 37 °C, 10 µl of the HIV-1 NL4-3 stock (Adachi et al.; 1986) was added to the cells. HIV-1 NL4-3 was derived by transient transfection of 293T cells with proviral DNA using the calcium phosphate method (BD Biosciences; CalPhos Mammalian Transfection Kit; # 631312). HIV-1 stocks were stored at -80 °C. 3 days post infection viral infection was detected using the β-Gal Screen Kit from Tropix (Biosystem; #T1027). The supernatant of cells was discarded and 40 µl of an 1:1 diluted β-Gal Screen solution containing the β-Gal substrate and PBS was added. After 30 min incubation, 35 µl of lysed cellular extracts were transferred into 96 well lumiplates (Nunc; #136101) and chemiluminescence was detected using a luminometer (OrionII, Berthold detection systems). β-galactosidase activities were obtained as relative light units per second (RLU/s). Values obtained in positive controls (infected cells containing no peptide) were set to 100% and all other values were calculated by dividing the absolute RLU/s in the presence of peptide by the median values obtained from cells containing no peptide x 100.

In the presence of fr. 27-29 from pool 4, reporter gene activities were increased compared to infected control cells containing no peptide (Fig. 1A). These results demonstrate that semen contains compound(s) that have favourable effects on HIV-1 infection. The presence of an HIV-1 activating peptide in human sperm is an important finding, since HIV-1 transmission in the human population preferentially occurs via sexual contact between HIV-1 infected and healthy sex partners.

To isolate the HIV-1 activating peptide, fractions 27-29 were further purified using chromatographic techniques.

### Second Reverse Phase Chromatography:

The third separation step was carried out on a reverse phase (RP) chromatography column (C18, 10 x 250 mm i.d., 5 microm particle size, 30 nm pore size, Vydac, Hesperia, CA, USA) (Figure 1B). The fractions 27-29 from pool 4 were applied to the RP C18 column and the peptides were eluated in gradient elution using following conditions:
Flow rate: 2 ml/min
Buffer A: 0.1 % TFA
Buffer B: A + 80 % (v/v) acetonitrile
Gradient elution: from 30% A to 60% B in 60 min
from 60% B to 100% B in 2 min

A total of 35 fractions of 2 ml were collected and aliquots of 1 % (20 microL) were lyophilized.
Aliquots corresponding to 1,3 ml sperm equivalent of the chromatography 011119-1 (1 % of the fraction) were then analyzed as described above. In the presence of peptide fraction 12 viral infectivities were increased 2 to 3 times compared to control cells (Fig. 1B).

### Third Reverse Phase Chromatography:

The fourth separation step was carried out on a reverse phase (RP) chromatography column (C4, 4 x 250 mm i.d., 5 microm particle size, 30 nm pore size, Phenomenex, Torrance, CA, USA) (Figure 1C). The fraction 12 from the second reverse phase chromatography was applied to the RP C4 column and the peptides were eluated in gradient elution using following conditions:
Flow rate: 0.8 ml/min
Buffer A: 0.1 % TFA
Buffer B: A + 80 % (v/v) acetonitrile
Gradient elution: from 28% A to 48% B in 60 min
from 48% B to 100% B in 1 min

A total of 30 fractions of 0.8 ml were collected and aliquots of 10 % (80 microL) were lyophilized.
Aliquots corresponding to 13 ml sperm equivalent of the chromatography 020228-2 (10 % of the fraction) were then analyzed in the virus inhibition assay exactly as described above. In the presence of peptide fractions 17+18 viral infectivities were increased 4 to 5 times compared to control cells (Fig. 1C).

### Example 2 - Biochemical analysis of HIV infection-activating peptide

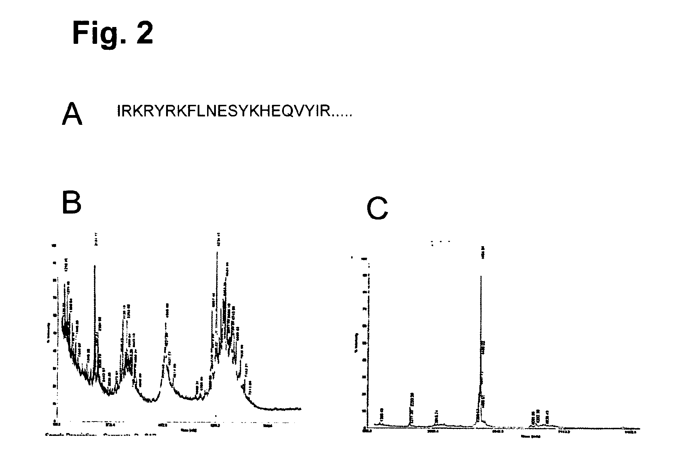

Mass analysis of the purified peptide and fractions was performed with Voyager De MALDI-TOF-MS (Applied Biosystems, Darmstadt, Germany) using standard conditions as recommended by the manufacturer (MALDI-MS; see Fig. 2). Mass determination of the synthetic peptide was additionally carried out on a Sciex API *III* quadrupol mass spectrometer (Sciex, Perkin-Elmer) with an electrospray interface. Peptides were sequenced on an 473 A gas-phase sequencer (Applied Biosystems, Weiterstadt, Germany) by Edman degradation with on-line detection of phenylthiohydantoin amino acids using the standard protocol recommended by the manufacturer. A database alignment (SwissProt database) of the obtained sequence (Fig. 2A) in connection with the molecular masses showed 100 % sequence identity to the precursor of human prostatic acid phosphatase (hPAP; accession number P15309), amino acid residues 85-120 of the hPAP precursor sequence (the numbering refers to the amino acids including the pro-sequence).
Interestingly, the major part of the isolated PAP85 peptide was glycosylated and showed molecular masses around 6500 Da (Fig. 2B). After deglycosylation of the peptide using the enzyme N-glycosidase F, the complete deglycosylated PAP85 peptide with a molecular mass of 4464 Da was obtained (Fig. 2C). Regarding this molecular mass information, the complete amino acid sequence of PAP85 comprised the residues 85-120 of the human PAP precursor with a calculated mass of 4464 Da. Our finding of a partially glycosylated PAP fragment in human sperm is consistent with earlier descriptions that the PAP precursor is glycosylated at residue asparagine 94 (N94).

The following fragment of PAP was detected in human sperm by mass and sequence determination:
Seq ID No. 1: PAP(85-120) IRKRYRKFLNESYKHEQVYIRSTDVDRTLMSAMTNL (Mr theor. 4464,1 Mr detected 4464.3)

### Example 3 - Chemical synthesis

Chemical synthesis of selected, linear PAP85 peptide in mg-amounts was carried out using conventional Fmoc chemistry. The synthesized peptides were purified using analytical reverse phase chromatography and their purity and identity were analyzed by mass and sequence determinations by the methods and equipments described above.

### Example 4 - PAP85-120 solutions form amyloid like fibrils

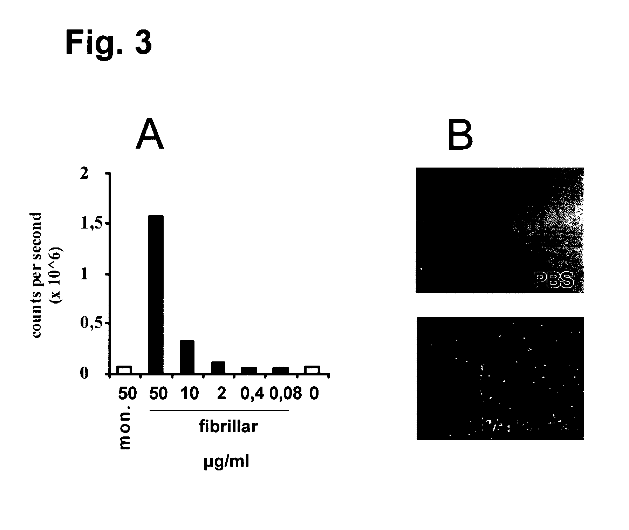

One of the most common techniques to identify the presence of amyloid fibrils is Thioflavin T (ThT) 1 fluorescence (Nilsson, 2004). ThT binding to PAP85-120 fibrils was assessed by first preparing a ThT stock solution by adding 8 mg ThT (Sigma) to 10mL phosphate buffer (10mM phosphate, 150mM NaCl, pH 7.0) and filtering this solution through a 0.2 µm syringe filter. Thereafter, this stock solution was 50 x diluted in PBS on the day of analysis (working solution). 10 µl samples (5 mg/ml) of either a freshly dissolved PAP85-120 solution or a PAP85-120 solution that was rotated on a table mixer at 37°C, 1200 rpm over night were then added to 1 ml of ThT working solution, mixtures were vortexed and fluorescence intensity was measured by excitation at 440 nm (bandwidth 4 nm) and emission at 482 nm (bandwidth 8 nm), averaging over 30 s using a Fluorolog II spectrofluorometer (SpexIndustries, Edison, NJ) as described (Nilsson, 2004). ThT binding assays revealed that the activated PAP85-120 solution contains amyloid like fibrils whereas freshly dissolved PAP85-120 does not contain detectable amounts of fibrils (Fig. 3A). Electron microscopic evaluation of an agitated PAP85-120 solution confirmed fibril formation (Fig. 3B). Thus, similarly to other amyloidogenic peptides, PAP85-120 forms fibrils after agitation.

### Example 5 - Amyloid like fibrils in activated PAP85-120 solutions enhance HIV infection

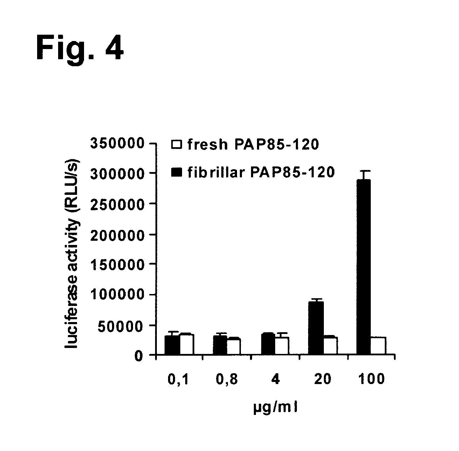

CEMX174 5.25 M7 cells, kindly provided by Nathaniel Landau, were cultivated in RPMI 1640 (Invitrogen; #21875,034) containing 10 % FBS and Pen/Strep. CEMX174 5.25 M7 cells contain LTR-luc (Firefly-luciferase) and LTR-GFP (green fluorescence protein) constructs stably integrated into the chromosomal DNA. After productive infection of CEMX174 5.25 M7 cells with HIV-1, HIV-2 or SIV, the viral transactivator protein Tat is expressed and transactivates the expression of Luc and GFP via interaction with the LTR. Thus HIV-1 infection can be detected by measuring luciferase activities in cellular lysates or GFP expression in living cells. HIV-1 stocks were generated by transient transfection of 293T cells with proviral DNA (see example 1).
PAP85-120 was dissolved in PBS (5 mg/ml) and either left untreated or incubated over night on a table rotator at 1300 rpm and 37°C. Incubation resulted in the formation of a turbid solution caused by PAP85-120 fibrils (see example 5). CEMX174 5.25 M7 cells were then infected with HIV-1 NL4-3 in the presence of either untreated PAP85-120 or the fibrillar peptide. 36 hrs post infection luciferase activities were determined in cellular lysates using the luciferase assay system (Promega; #E1501). Briefly, cells were resuspended, transferred to 96 well V shaped plates (Greiner; #651180), and centrifuged for 5 min at 1200 rpm. Cell supernatants were removed and remaining cell pellets were lysed in 30 µl 1x luciferase lysis buffer (Promega; #E1531). After transfer of 20 µl of the lysates to 96 well lumiplates (Nunc; #136101), 100 µl of luciferase reagent (Promega) was added and chemiluminescence was detected using a luminometer (see example 1), detecting luciferase activities as relative light units per second (RLU/s). Average values of infectivity derived from triplicate infections are shown relative to those measured in the absence of peptides (100%). Results shown in Fig. 4 demonstrate that only the fibrillar PAP85-120 solution dose dependently enhanced HIV-1 infection whereas the freshly dissolved PAP85-120 solution had no effect. 50 µg/ml of fibrillar PAP85-120 enhanced HIV-1 infection ∼ 10 fold.

### Example 6 - PAP85-120 fibrils are non cytotoxic.

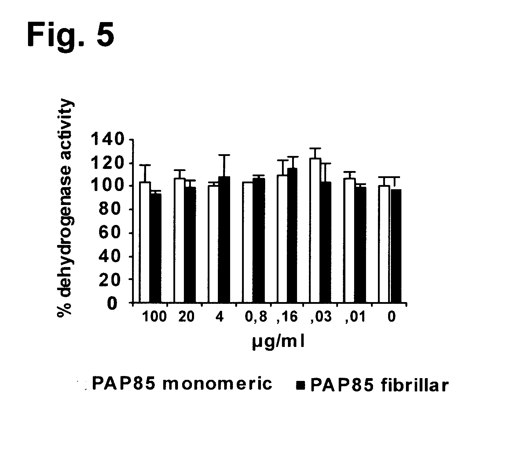

To determine possible cytotoxic effects of PAP85-120 fibrils, serial dilutions of fibrils were added to stimulated peripheral blood mononuclear cells (PBMC) and incubated for 3 days (see example 8). Thereafter 10 µl of a 5 mg/ml MTT (Sigma; # M2128) solution was added and cells were incubated for 4 h at 37°C. In living cells mitochondrial dehydrogenases oxidize the yellow MTT salt to blue formazan crystals (Schiff et al.; 1985). After dissolving the crystals in 1:1 Ethanol/DMSO, samples containing living cells appear blue whereas samples from dead cells are clear. Optical density (OD) was measured at 560/650 nm using a photometer (Molecular Devices). As shown in Fig. 5, cell viability in samples containing high concentrations of fibrillar PAP85-120 was not affected by the peptide indicating that the peptide neither exerted cytotoxic effects nor induced cellular proliferation.

### Example 7 - Fibrillar PAP85-120 enhances HIV-1 infection independently of the HIV-1 coreceptor tropism

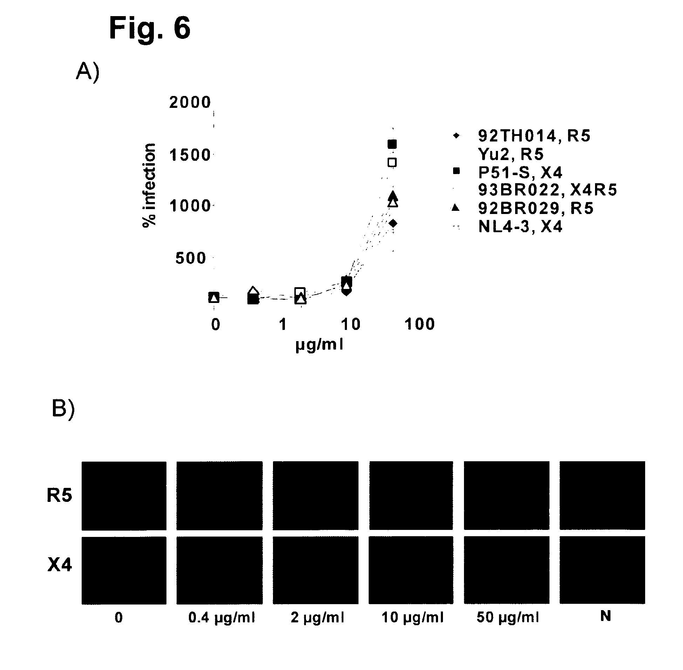

To productively infect a target cell, HIV-1 requires the primary CD4 receptor and a second molecule on the cell surface, one of the coreceptors CCR5 or CXCR4 (Chantry et al.; 2004). Previous data were obtained with HIV-1 NL4-3 which is strictly CXCR4 (X4) tropic (Papkalla et al.; 2002). To analyze the enhancing effect of fibrillar PAP85-120 on HIV-1 coreceptor usage, CXCR4 (X4), CCR5 (R5) and dual tropic HIV-1 NL4-3 recombinants (Papkalla et al., 2002) were produced by transient transfection of 293T cells. CEMX174 5.25 M7 cells were then infected with 0,1 ng of p24 antigen in the presence of fibrillar PAP85-120 and 3 dpi luciferase activities were determined as described above. PAP85-120 fibrils enhanced infection of all HIV-1 variants analysed independently of the coreceptor tropism (Fig. 6A). For example, in the presence of 50 µg/ml PAP85-120 the R5 tropic 92BR029 HIV variant was enhanced 8.2 fold and X4 tropic P51-S up to 15.9 fold. UV microscopic evaluation of CEMX174 5.25 M7 cells infected with either X4 tropic HIV-1 NL4.3 or the R5 tropic 92TH014-2 variant confirmed that PAP85-120 fibrils dose dependently activated HIV-1 infection. For example, the amount of GFP expressing cells (see example 5) in infected controls containing no fibrils is close to that of uninfected cells. However, in the presence of 2 µg/ml and higher concentrations of fibrils, the number of infected (GFP expressing) cells is markedly increased. Thus, PAP85-120 fibrils enhance HIV-1 infection independently of the coreceptor tropism.

### Example 8 - PAP85-120 promotes HIV-1 infection of PBMC and macrophages independently of the coreceptor usage.

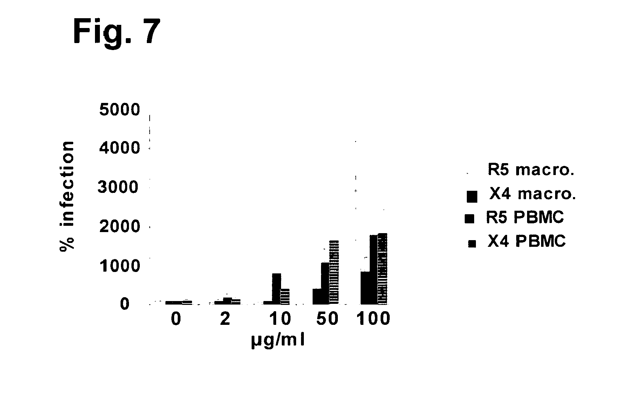

To analyze whether PAP85-120 also promotes infection of primary cells with X4 and R5 tropic HIV-1, experiments in peripheral blood mononuclear cells (PBMC) were performed. PBMC were obtained from whole blood using Ficoll density gradient centrifugation. Purified PBMC were stimulated for 3 days in RPMI-1640 medium containing 20% FBS, 10 ng/ml IL-2 (Strathmann) and 3 µg/ml phytohemagglutinine (Oxoid; # 30852801). Then, PBMC were sedimented by centrifugation at 1200 rpm and resuspended in RPMI1640 (10%FBS, 10 ng/ml IL-2). Next, 5 x 10⁵ cells were sown out in a volume of 50 µl RPMI and 10 µl of PAP85-120 fibril dilutions were added. Subsequently cells were infected with 0.5 and 5 ng p24 antigen of the R5 tropic HIV-1 92TH014-2 clone (a) (Papkalla et al.; 2002) and the X4 tropic HIV-1 NL4-3 Luc (He at al.; 1995) (b) in a total volume of 100 µl. Both viruses were obtained by transient transfection of 293T cells and contain the luciferase gene instead of nef. Three days post infection cells were pelleted and lysed in 30 µl luciferase lysis buffer. Luciferase activity was detected using 20 µl of lysates and 100 µl of luciferase assay reagent. The increase of luciferase activities with increasing concentrations of peptide demonstrate that fibrillar PAP85-120 dose dependently promoted infection of PBMC by R5 and X4 tropic viruses (Fig. 7). For example, 100 µg/ml fibrils enhanced HIV-1 infection 20 fold. To analyze the effect of fibrillar PAP85-120 on HIV-1 infection of macrophages, 5 x 10⁵ PBMC were sown out in 500 µl RPMI (10% FBS, 10 ng/ml GM-CSF) in 48 well dishes (Becton-Dickinson; # 353078). After 5 days non-adherent cells were carefully washed out and 400 µl of fresh medium containing GM-CSF was added. Two days later medium was removed, 130 µl of fresh medium and 20 µl of fibrillar PAP85-120 dilutions were added to the differentiated macrophages and then infected with the indicated amounts of the X4 tropic NL4-3 Luc (a) and the R5 tropic 92TH014-2 Luc (b) in a total volume of 200 µl. Virus infection was detected after 3 days using the Luciferase assay reagent. As shown in Fig. 7, PAP85-120 also enhanced HIV-1 infection of macrophages independently of the coreceptor used. Overall infection rates of the X4 tropic NL4-3 were reduced compared to those obtained with the R5 tropic (Fig. 7) variant probably because of the lack of CXCR4 expression in macrophages.

### Example 9 - PAP85-120 fibrils enhance HIV infection independently of the HIV-1 geno- or subtype

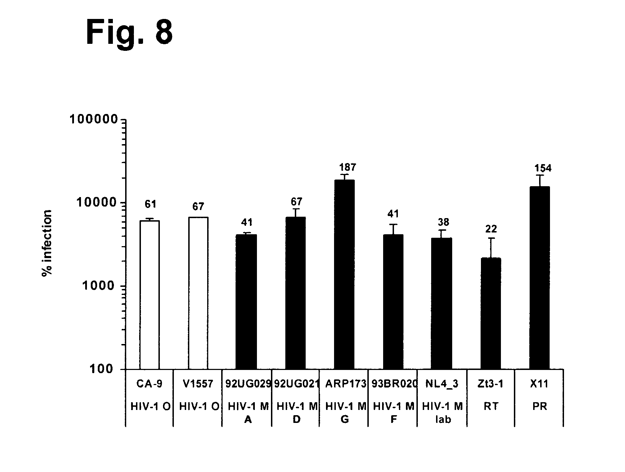

To detect the enhancing effect on infection by several HIV-1 variants, fibrillary PAP85-120 dilutions were added to CEMX174 5.25 M7 cells and infected with HIV-1 group O isolates (Dittmar et al.; 1999) and some HIV-1 subtypes (obtained from the NIH AIDS research and reference program). Luciferase activities obtained two days post infection revealed that PAP85-120 enhanced infection of all HIV-1 O and HIV-1 M strains as well as two drug resistant HIV-1 NL4-3 derivatives tested. For example, 50 µg/ml fibrils enhanced the infection rate of the HIV-1M subtype G isolate ARP173 from 100 % (control containing now peptide) to 18,700 %. Data shown in Fig. 8 demonstrate that PAP85-120 fibrils have a broad HIV-1 enhancing activity.

### Example 10. PAP85-120 lowers the threshold of a productive HIV-1 infection.

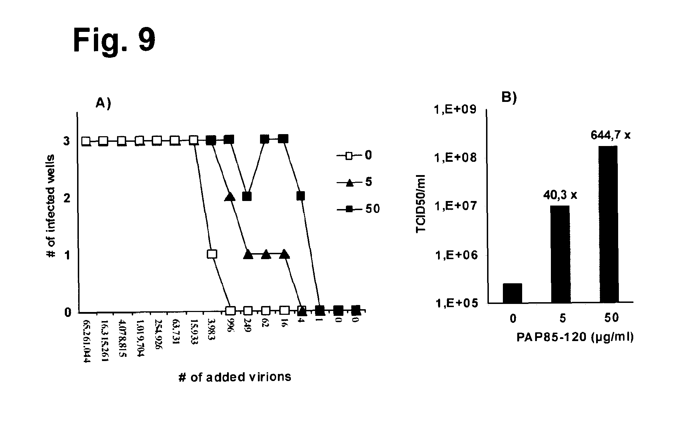

The magnitude of HIV-1 entry enhancement in infectivity assays may underestimate the real potency of PAP85-120 fibrils because 0.5 to 5% of the target cells already became infected in its absence. To determine the effect of PAP85-120 fibrils on the TCID50 (50% tissue culture infectious dose) of virus stocks more accurately, we performed thorough limiting dilution infection assays. The TCID50 (50% tissue culture infectious dose) was determined and calculated as described by the ACTG Laboratory Technologist Committee (http://aactg.s-3.com/pub/download/labmanual/43-ALM-TCID50-Determination.pdf). R5 tropic 92TH014-2 virus was generated by transfection of 293T cells with low concentrations of provirus to prevent cell lysis and release of unassembled p24. After overnight incubation, cells were washed with DMEM (10% FCS) and virus stocks were harvested 32 hrs later. The p24 antigen concentrations were determined using the HIV-1 p24 Antigen Capture Assay Kit (NIH AIDS Reagent Program). To quantify the infectious titer, serial four-fold dilutions of virus stocks mixed with fibrillar PAP85-120 or PBS were made in 96-well flat-bottomed tissue culture plates. After 10 min, 2x10⁵ PHA/IL-2 stimulated PBMCs were added to each well. Every two days, half of the supernatants was removed and frozen and fresh medium was added. 8 dpi PBMC supernatants were examined for virus production using a p24 antigen ELISA. Cells were considered to be productively infected when >2 ng/ml p24 was detected in the cell-free supernatant (background <0.10 ng/ml, n=24). The number of virions in the virus stocks were calculated assuming that there are 5000 p24 CA proteins per viral particle (Briggs et al., 2004).
Treatment with PAP85-120 fibrils enhanced the TCID50 of HIV-1 in PBMC by 40 x (5 µg/ml) or 645 x (50 µg/ml) (Fig. 9B). Notably, 50 µg/ml of fibrillar PAP85-120 allowed the detection of even a few single viruses present in the original stock solution (Fig. 9A). In the absence of fibrils the ratio of detectable infectious units to 1 virus particles was on the order of 1:1,000 to 1:10,000, which is in agreement with published data (Dimitrov et al., 1993; Rusert et al., 2004). Recent data suggest that the apparent high frequency of "non-infectious" HIV-1 particles is largely due to the low frequency of successful virus-cell interactions (Thomas et al., 2007). In agreement with this possibility, our data demonstrate that a few HIV-1 particles are sufficient for spreading infection in the presence of an effective attachment factor. The presence of an HIV activating peptide in human sperm could have great implications in the natural transmission of HIV-1 during sexual intercourse. The HIV-1 transmission rate per sexual contact is lower than 0.1 %.

### Example 11 - PAP85-120 fibrils are general activators of retroviral infection

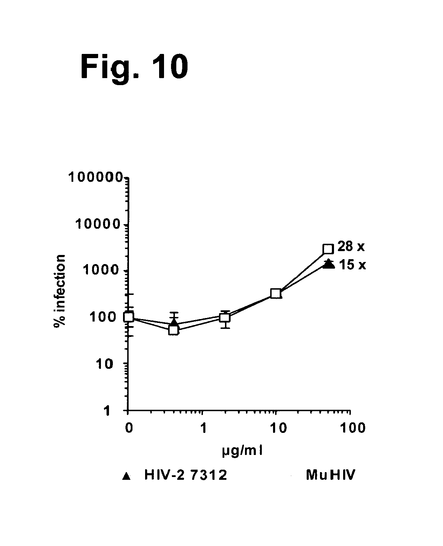

To analyze the effect of fibrillar PAP85-120 on HIV-2 as well as Mouse leukemia virus we generated virus stocks by transient transfection of 293T cells. The HIV-2 7312 was obtained from Beatrice Hahn (University of Alabama at Birmingham, USA) and MuHIV, a mouse leukemia virus encoding the HIV-1 envelope from Klaus Überla (Ruhr University of Bochum, Germany). The amount of virus was detected using reverse transcriptase assay (Potts, 1990) and virus stocks were then adjusted to contain similar RT activities. 10 x dilutions thereof were used to infect TZMbl cells in the presence of fibrillar PAP85-120 and viral infection was determined 3 days post infection using the β-galactosidase assay. As shown in Fig. 10, PAP85-120 fibrils dose dependently enhanced MuHIV and HIV-2 infection. These results show that the enhancing effect of PAP85-120 fibrils is not restricted to HIV-1.

### Example 12 - PAP85-120 fibrils infection of lentiviral core particles harbouring glycoproteins derived from VSV-G and MLV

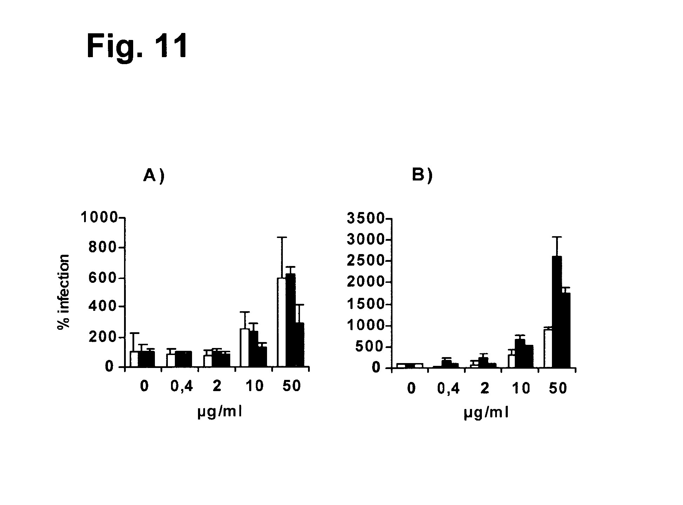

HIV-1 pseudotyped particles were generated by cotransfection of 1) a pBRHIV-1 NL4-3 derivative containing a deletion in the envelope and carrying luciferase reporter gene instead of *nef* and 2) an expression plasmid encoding the glycoprotein of the vesicular stomatitis virus (VSV-G) or the Mouse leukemia virus (MLV env). Virus stocks were generated as described above and used to infect TZM-bl cells in the presence of PAP85-120 fibrils. Infection rates obtained after three days demonstrate that fibrillar PAP85-120 dose dependently enhanced infection by VSV-G (Fig 11A) and MLV (Fig. 11B) pseudotyped particles up to 6 fold (VSV-G) or 25 fold (MLV), respectively. Pseudoparticles carrying the MLV or VSV-G envelope glycoproteins are widely used to enhance transduction efficiencies in gene transfer experiments. Thus, PAP85-120 fibrils might further enhance gene transfer efficiencies.

### Example 13 - PAP85-120 fibrils enhance Hepatitis C virus infection.

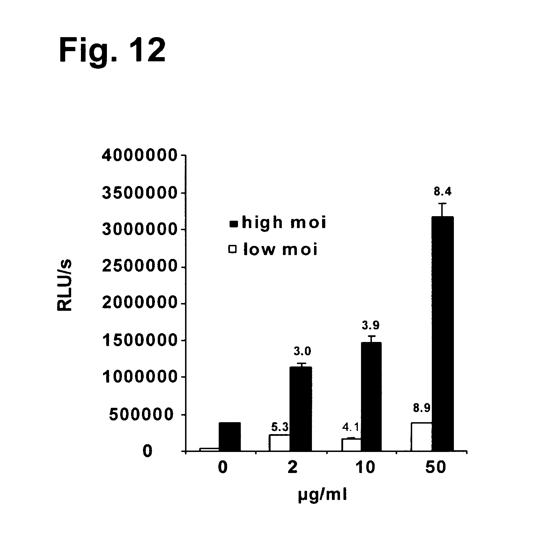

HCV reporter viruses (Luc-Jc1) were generated as described recently (Koutsoudakis et al., 2006). Briefly, culture fluid of Huh7 cells transfected with HCV luciferase reporter virus RNA was harvested 48 h post transfection, cleared by passing through 0.45 µm pore size filters, and used directly for infection assays. Huh7 target cells were seeded at a density of 4x10⁴ per well of a 12 well plate 24 h prior to inoculation with 500 µl virus preparation in the presence or absence of stimulatory peptides at the concentration given in the figure. Inoculation was terminated by aspiration of virus-containing medium and addition of fresh culture medium 12 hours later. Infection was quantified by assessing the amount of luciferase reporter activity per well 72 h post inoculation. All assays were done in duplicate, mean values of duplicates and the respective error ranges are given. Results shown in Fig. 12 demonstrate that fibrillar PAP85-120 dose dependently enhanced HCV infection.

### Example 14 - PAP85-120 fibrils enhance xenotropic murine-like retrovirus (XMRV) infection

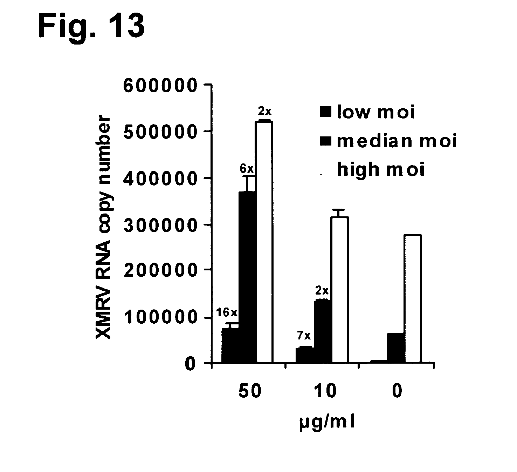

DU145 cell lines (from ATCC) were cultured in RPMI 1640 media supplemented with 10% fetal bovine serum. To isolate virus, the cell culture supernatants of chronically XMRV (strain VP62)-infected LNCaP cells at 10 or 13 day post-transfection were passed through a 0.2 µm filter (Whatman) and stored at - 70°C. Virus infections were performed in triplicate in 12-well plates using cells plated one day prior to infection. Cells were at about 50% confluency at the time of infection. The cell media were replaced with 100 µl of media containing 10 or 50 µg per ml of PAP peptide or PBS and VL, L and H = 1/5000, 1/100, and 1/10 dilutions of xenotropic murine-like retrovirus (XMRV) stock and incubated for 3 h to allow virus adsorption. Cells were washed once with PBS and fresh media containing fetal bovine serum were then added to the cells. RNAs were prepared from cells using Trizol method according to manufacturer's instruction. RNA concentrations were measured using NanoDrop® ND-1000 UV-Vis Spectrophotometer and 100 ng of RNA were used for XMRV qRT/PCR using Ag-Path kit with XMRV Env primers and probe. To normalize gene expression level, pre-developed TaqMan Assay Reagents for human GAPDH gene (cat: 4333764T) were used. Human GAPDH primers and probe were added to each reaction and relative CT of both XMRV env and GAPDH were determined using qRT/PCR. To normalize the expression level of env gene, the 2-CT (Livak) method was used. As demonstrated in Fig. 13 PAP85-120 enhanced XMRV infection up to 16 fold when a low amount of virus was used for infection. Using a high multiplicity of infection the effect of PAP85-120 was low probably due to the already high infection rates in samples containing no fibrils.

### Example 15 - PAP85-120 increases HIV-1 attachment to cells

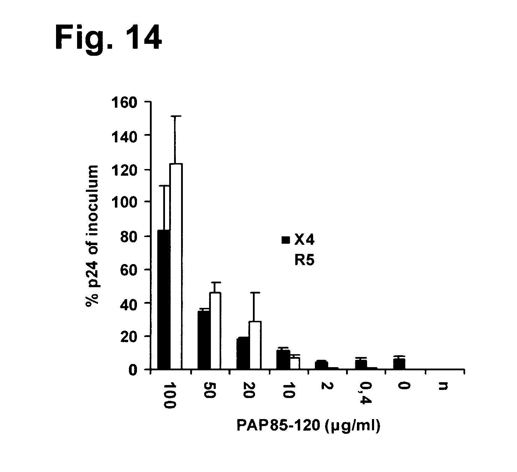

To analyze whether PAP85-120 fibrils enhance attachment of HIV-1 to cells, TZMbl cells were plated in 100 µl 96 well plates, the day after 20 µl PAP85-120 dilutions were added and subsequently infected with 80 µl of the X4 tropic HIV-1 NL4.3 (7.5 ng p24 antigen) and the R5 tropic 92TH014-2 variant (2,5 ng p24 antigen). Final PAP85-120 concentrations were 100, 50, 20, 10, 2, 0.4 and 0 µg/ml. After 4 hrs incubation at 37°C supernatants were removed, cells were washed two times in PBS and cells and cell associated virus was lysed in 200 µl 1% Triton. The amount of cell associated virus was detected by p24 antigen ELISA. In the presence of 100 µg/ml PAP85-120 fibrils almost all input virus could be recovered, indicating that PAP85-120 fibrils allowed attachment of all viral particles present in the inoculum also explaining the drastic enhancing effects of Pap85-120 fibrils in limiting virus dilution experiments (see example 10) (Fig. 14).

### Example 16 - Fibrils are responsible for the HIV activating activity of agitated PAP85-120 solutions.

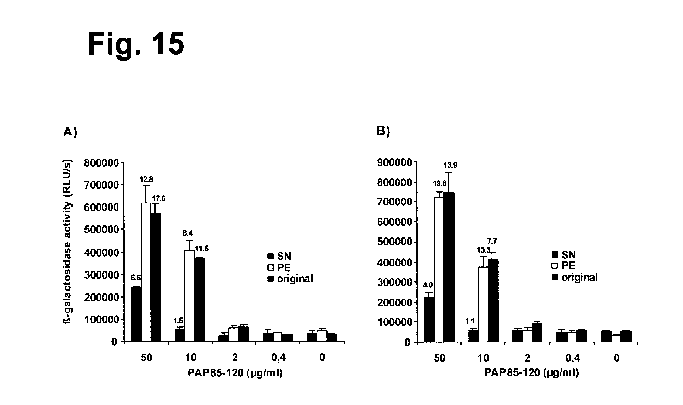

To analyze whether the fibril fraction or the soluble fraction of a fibrillar PAP85-120 solution is responsible for the HIV enhancing effect, an agitated fibril containing PAP85-120 solution was centrifuged 10 min at 14000 rpm, the clear supernatant was removed and the remaining pellet resuspended in 600 µl PBS. Thereafter, 10 µl of serial 5 fold dilutions of the supernatant (SN), the resuspended pellet (PE) or the original solution (original) were added to 4000 TZMbl cells (50 µl) and infected with equal infectious doses of the X4 tropic HIV-1 NL4.3 or the R5 tropic 92Th014-2 variant (40 µl virus dilution in medium). Final concentrations of PAP85-120 solutions were 50, 10, 2, 0.4 and 0 µg/ml. After one day 100 µl fresh medium was added and infection rates were measured 3 days post infection using the GalScreen assay. As shown in Fig. 15A and B, the PAP85-120 pellet and the original solution enhanced X4 and R5 HIV-1 infection with similar efficiencies. 50 µg/ml PAP85-120 supernatant enhanced HIV-1 infection as well, however, with reduced activity compared to the PAP85-120 pellet or the original solution. These data indicate that the majority of the enhancing effect of agitated PAP85-120 solutions on HIV infection is mediated by precipitable fibrils, but also the soluble, non precipitable fraction contains enhancing activity, probably low weight protofilaments or proto fibrils that cannot be pelleted under the chosen conditions (Fig. 15).

### Figure legends:

**Figure 1****: Purification of PAP85 from sperm.** For details see examples 1 and 2. **A)** Reversed phase (RP) chromatography of sperm peptides previously extracted by acetic acid and enriched by cation exchange chromatography. Exemplary shown is the preparative fractionation of extract pool 4 obtained from 130 ml of human whole sperm. HIV activating fractions 27-29 are marked and were consecutively purified by further RP chromatographic steps **B)** The pooled fractions 27-29 from A were subjected to a further RP-HPLC and the resulting fractions were assayed for anti- or proviral activity. P4-CCR5 cells were infected with HIV-1 NL4-3 in the presence or absence of peptides. Virus infectivity was determined by measuring the β-galactosidase activities in the cellular extracts at 3 days post-infection using the Gal Screen Kit from Tropix. The shaded area of the chromatogram in the inlet specifies the active fraction. **C)** Active fraction 12 from B was further purified by RP-HPLC and resulting fractions were tested as described above. HIV-1 promoting fractions 17+18 was analyzed using sequence and mass spectroscopic analysis. Average values (±SD) of infectivities derived from triplicate infections are shown relative to those measured in the absence of peptides (100%). Controls show non infected cells.

**Fig. 2****. Analysis of PAP85 from semen.** Sequence and mass spectrometric analysis of fractions 17+18 from 1C revealed the isolation of the PAP85 fragment PAP 85-120. **A)** The first sequence analysis by Edman degradation revealed a peptide sequence starting at sequence position 85 of the human prostatic acid phosphatase (PAP). **B)** Interestingly, mass spectrometric analysis (MALDI-MS) of the sequenced fraction showed variously glycosylated and non-glycosylated isoforms of the peptide starting with the PAP85 sequence in the molecular mass range of 6500 and 4500 Da. **C)** After deglycosylation of the fraction using the enzyme N-glycosidase F, a unique, completely deglycosylated PAP85 peptide with a molecular mass of 4464 Da was obtained. Regarding this molecular mass information, the complete amino acid sequence of PAP85 comprised the residues 85-120 of the human PAP precursor with a calculated mass of 4464 Da. Our finding of a partially glycosylated PAP fragment in human sperm is consistent with earlier descriptions that the PAP precursor is glycosylated at residue asparagine 94 (N94).

**Fig. 3****. Fibril formation by PAP85-120. A)** 10 µl samples of either a freshly dissolved PAP85-120 solution or a PAP85-120 solution that was rotated on a table mixer at 37°C, 1200 rpm over night were added to 1 ml of a Thioflavin T working solution, mixtures were vortexed and fluorescence intensity was measured by excitation at 440 nm (bandwidth 4 nm) and emission at 482 nm (bandwidth 8 nm), averaging over 30 s using a Fluorolog II spectrofluorometer (SpexIndustries, Edison, NJ) as described (Nilsson, 2004). mon.; monomeric. **B)** Electron micrographs of agitated PBS and a agitated PAP85-120 solution.

**Fig. 4****: Fibrillar PAP85-120 solutions enhance HIV-1 infection.** PAP85-120 was dissolved in PBS (5 mg/ml) and either left untreated or incubated over night on a table rotator at 1300 rpm and 37°C. Incubation resulted in the formation of a turbid solution caused by PAP85-120 fibrils. CEMX174 5.25 M7 cells were then infected in the presence of either the untreated PAP85-120 solution or the fibrillar peptide with HIV-1 NL4-3. 36 hrs post infection luciferase activities were determined in cellular lysates. Values represent average values derived from triplicate infection +/- SD. RLU/s: relative light units per second.

**Fig. 5****: PAP85 is non cytotoxic.** Peripheral blood mononuclear cells (PBMC) were stimulated with phytohemagglutinine (2 µg/ml) and Interleukin 2 (10 µg/ml) for three days. Thereafter cells were sown out in 96 well dishes containing the indicated concentrations of freshly dissolved monomeric PAP85 or fibrillar PAP85 and incubated for 3 days. Cytotoxicity was determined by detecting dehydrogenase activity in living cells using the MTT assay. Values represent average values +/- SD derived from triplicates. Deydrogenase activities obtained from samples containing no peptide were set to 100%.

**Fig. 6****. Fibrillar PAP85-i20 enhances HIV-1 infection independently of coreceptor usage. A)** CEMX174 5.25 M7 cells were infected with 0,1 ng p24 antigen of indicated HIV-1 NL4_3 recombinants (Papkalla et al., 200x) in the presence or absence of PAP85 fibrils. Infection rates were determined 3 days post infection by detecting virus induced luciferase activity. Data represent average values +/- SD obtained from triplicates. Luciferase activities in infected cells containg no peptide were set to 100%. Abbreviations: R5, CCR5 tropic, R5: CXCR4 tropic, X4R5: dual tropic. **B)** Microscopic examination of cells infected with X4 tropic HIV-1 NL4-3 or the R5 tropic 92Th014 V3 recombinant virus in the presence of absence of PAP85-120 fibrils. Fibrils were added to CEMX174 5.25 M7 cells and subsequently infected. After 2 days, cells were pelleted, fixed with 50 µl 2% paraformaldehyde and examined using UV microscopy. N; uninfected.

**Fig. 7****. Fibrillar PAP85 favours HIV-1 infection of macrophages and PBMC**. Cells were infected with 0.5 and 5 ng of luciferase encoding X4 tropic HIV-1NL4_3 or the R5 tropic 92TH014.12 derivative (Papkalla et al., 2002) in the presence or absence of indicated PAP85 concentrations. 3 days post infection virally encoded luciferase was detected in cell lysates using a chemiluminescence assay. Values represent average values derived from 0.5 and 5 ng infections (each performed in triplicate) +/- SE. Luciferase activities obtained from samples containing no peptide were set to 100%.

**Fig. 8****. PAP85-120 fibrils enhance HIV-1 infection independently from subtype.** CEMX174 5.25 M7 cells were infected with primary HIV-1 stocks in the presence of 50 µg/ml PAP85-120 fibrils and infection was determined 2 days post infection. Values obtained from controls containing no peptide were set to 100%. Abbreviations: HIV-1 O: outlier; HIV-1 M: major; A,D,G,F indicate HIV-1 subtype; lab: laboratory adapted molecular HIV-1 clone; ZT3-1 and X11, Reverse transcriptase resistant or protease resistant HIV-1 clones.

**Fig. 9****. Fibrillar PAP85-120 lowers the threshold required for productive infection. (A)** Limiting dilution analysis of HIV-1. PBMC were infected in triplicate with 4-fold dilutions of an R5 tropic HIV-1 NL4-3 virus stock in the presence of the indicated concentrations of fibrillar PAP85-120. Indicated is the number of cultures that became productively infected 8 days post infection. **(B)** Effect of PAP85-120 on the TCID50 of HIV-1. TCID50 values were calculated based on the data shown in panel A.

**Fig. 10****. Fibrillar PAP85-120 is a general enhancer of retroviral infection.** HIV-2 and MuHIV virus stocks were generated by transient transfection of 293T cells and virus amount was quantified in Reverse transcriptase (RT) assay. Virus stocks were adjusted to similar RT activities and 10 fold dilutions thereof were used to infect TZMbl cells in the presence or absence of peptide. 3 dpi infcetion rates were determined by β-galactosidase assay. Shown are average values obtained derived from triplicates +/- SD. Values obtained from infected controls containing no peptide were set = 100%.

**Fig. 11****. PAP85-120 fibrils enhance VSVpp and MLVpp infection.** Pseudoparticles were produced by cotransfection of an env deleted luciferase encoding HIV-1 proviral construct and expression plasmids encoding the G protein of VSV **(A)** or the envelope glycoprotein of MLV **(B).** 293T cells were infected with pure virus (white bars), or 10 x (black) or 100 x (grey) dilutions of virus stock. VSV-G and MLV env mediated infection was analyzed after three days by detecting luciferase activities in cellular lysates. Shown are average values +/- SD derived from triplicate infections. Reporter activities in control infections containing no fibrils: 100%.

**Fig. 12****. PAP85-120 fibrils favour Hepatitis C Virus infection. HUH7 cells** were incubated with indicated amounts of PAP85-120 fibrils and subsequently infected with a low and a high multiplicity of infection (moi) using an luciferase expressing Hepatitis C Virus genotype 1a construct. 3 days later cells were lysed and luciferase activity was detected by chemiluminescence.

**Fig. 13****. PAP85-120 fibrils enhance XMRV infection.** DU145 cells, a prostate cell line, were infected with high, median and low moi of the xenotropic murine-like retrovirus, a retrovirus isolated from human prostate cancer in the presence or absence of fibrillar PAP85-120. 3 days later XMRV RNA amounts were detected by quantitative real time PCR.

**Fig. 14****. PAP85-120 fibrils enhance HIV-1 cell attachment.** TZMbl cells were inoculated with X4 and R5 tropic HIV-1 in the presence or absence of PAP85-120 fibrils. After 4 hrs the inoculum was removed, cells were washed twice and lysed in 1% Triton. Cell associated virus was measured using a p24 antigen ELISA. Values are normalized to the total amount of input virus (100%).

**Fig. 15****. The precipitable fraction of an agitated PAP85-120 solution contains the majority of the HIV enhancing activity. An** fibril containing PAP85-120 solution was centrifuged at 14000 rpm, the supernatant taken and the pellet resuspended in the original volume of PBS. Serial dilutions of the resuspended pellet, the supernatant and the original solution were added to TZMbl cells and infected with X4 **(A)** and R5 **(B)** tropic HIV-1. After 3 days infection rates were determined by GalScreen. SN, supernatant; PE, pellet;

### Literature:

Adachi A, Gendelman HE, Koenig S, Folks T, Willey R, Rabson A, Martin MA. Production of acquired immunodeficiency syndrome-associated retrovirus in human and nonhuman cells transfected with an infectious molecular clone. J Virol. 1986 Aug;59(2):284-91. Chantry D. HIV entry and fusion inhibitors. Expert Opin Emerg Drugs. 2004 May;9(1):1-7. Review. Charneau P, Borman AM, Quillent C, Guetard D, Chamaret S, Cohen J, Remy G, Montagnier L, Clavel F. Isolation and envelope sequence of a highly divergent HIV-1 isolate: definition of a new HIV-1 group. Virology. 1994 Nov 15;205(1):247-53. Choe BK, Pontes EJ, Dong MK, Rose NR. Double-antibody immunoenzyme assay for human prostatic acid phosphatase. Clin Chem. 1980 Dec;26(13):1854-9. Coffey DS, Pienta KJ. New concepts in studying the control of normal and cancer growth of the prostate. Prog Clin Biol Res. 1987;239:1-73. Review. Cusan L, Gomez JL, Dupont A, Diamond P, Lemay M, Moore S, Labrie F. Metastatic prostate cancer pulmonary nodules: beneficial effects of combination therapy and subsequent withdrawal of flutamide. Prostate. 1994 May;24(5):257-61 Dimitrov DS, Willey RL, Sato H, Chang LJ, Blumenthal R, Martin MA. Quantitation of human immunodeficiency virus type 1 infection kinetics. J Virol. 1993 Apr;67(4):2182-90. Dittmar MT, Zekeng L, Kaptue L, Eberle J, Krausslich HG, Gurtler L. Coreceptor requirements of primary HIV type 1 group O isolates from Cameroon. AIDS Res Hum Retroviruses. 1999 May 20;15(8):707-12. Griffiths JC. Prostate-specific acid phosphatase: re-evaluation of radioimmunoassay in diagnosing prostatic disease. Clin Chem. 1980 Mar;26(3):433-6. Koutsoudakis, G., A. Kaul, E. Steinmann, S. Kallis, V. Lohmann, T. Pietschmann, and R. Bartenschlager. 2006. Characterization of the early steps of hepatitis C virus infection by using luciferase reporter viruses. J. Virol. 80:5308-5320.
Münch J., Rücker E., Ständker L., Adermann K., Goffinet C., Schindler M., Wildum S., Chinnadurai R., Rajan D., Giménez-Gallego G., Fowler D.M., Koulov A., Kelly J.W., Keppler O.T., Forssmann W.-G., Kirchhoff F. (2007). Semen-derived Amyloid Fibrils Drastically Enhance HIV infection. Cell, Dec 14. Nilsson MR. Techniques to study amyloid fibril formation in vitro. Methods 2004, 34, 151-160. Papkalla A, Munch J, Otto C, Kirchhoff F. Nef enhances human immunodeficiency virus type 1 infectivity and replication independently of viral coreceptor tropism. J Virol. 2002 Aug;76(16):8455-9. Potts B.J.. "Mini" reverse transcriptase (RT) assay, pp. 103-106. In A. Aldovini, and B.D. Walker (ed.), Techniques in HIV research. Stockton Press, New York, N.Y. 1990 Rusert P, Fischer M, Joos B, Leemann C, Kuster H, Flepp M, Bonhoeffer S, Günthard HF, Trkola A. Quantification of infectious HIV-1 plasma viral load using a boosted in vitro infection protocol. Virology. 2004 Aug 15;326(1):113-29. Schiff D, Chan G, Poznansky MJ. Bilirubin toxicity in neural cell lines N115 and NBR10A. Pediatr Res. 1985 Sep;19(9):908-11. Sharief FS, Li SS. Structure of human prostatic acid phosphatase gene. Biochem Biophys Res Commun. 1992 May 15;184(3):1468-76 Shaw LM, Yang N, Brooks JJ, Neat M, Marsh E, Seamonds B. Immunochemical evaluation of the organ specificity of prostatic acid phosphatase. Clin Chem. 1981 Sep;27(9):1505-12. Thomas JA, Ott DE, Gorelick RJ. Efficiency of human immunodeficiency virus type 1 postentry infection processes: evidence against disproportionate numbers of defective virions. J Virol. 2007. 81. 4367-70. Vihko P, Kontturi M, Korhonen LK. Purification of human prostatic acid phosphatase by affinity chromatography and isoelectric focusing. Part I. Clin Chem. 1978 Mar;24(3):466-70. Vihko P. Characterization of the principal human prostatic acid phosphatase isoenzyme, purified by affinity chromatography and isoelectric focusing. Part II. Clin Chem. 1978 Oct;24(10):1783-87. Vihko P, Kostama A, Janne O, Sajanti E, Vihko R. Rapid radioimmunoassay for prostate-specific acid phosphatase in human serum. Clin Chem. 1980 Oct;26(11):1544-7. Vihko P, Lukkarinen O, Kontturi M, Vihko R. Effectiveness of radioimmunoassay of human prostate-specific acid phosphatase in the diagnosis and follow-up of therapy in prostatic carcinoma. Cancer Res. 1981 Mar;41(3):1180-3. Vihko P, Virkkunen P, Henttu P, Roiko K, Solin T, Huhtala ML. Molecular cloning and sequence analysis of cDNA encoding human prostatic acid phosphatase. FEBS Lett. 1988 Aug 29;236(2):275-81.

Vihko P, Kurkela R, Porvari K, Herrala A, Lindfors A, Lindqvist Y, Schneider G. Rat acid phosphatase: overexpression of active, secreted enzyme by recombinant baculovirus-infected insect cells, molecular properties, and crystallization. Proc Natl Acad Sci USA. 1993 Feb 1;90(3):799-803.
Yam LT, Janckila AJ, Li CY, Lam WK. Presence of "prostatic" acid phosphatase in human neutrophils. Invest Urol. 1981 Jul;19(1):34-8.

## Claims

1. A peptide consisting of a partial sequences of the 51 residue section of amino acids 80-130 of the human prostatic acid phosphatase (hPAP) amino acid sequence, in particular amino acid sequence SEQ ID No. 1 .

2. A peptide, which is any fragment of amino acid residues 80-130 of the human prostatic acid phosphatase, wherein the fragment promotes infection of a cell with a virus, and the fragment preferably consists of at least 12, more preferably 20 amino acids.

3. The peptide of claim 1 or 2 with at least one of the following modifications:
- a deletion of amino acids at the N- and/or C-terminus, wherein the peptide consists of at least 20 amino acids,
- addition of up to 10 amino acids at the N- and/or C-terminus,
- amino acid exchange of up to 5 amino acids,
- amino acid insertion or deletion within the sequence of up to 3 amino acids,
wherein the modified peptide promotes infection of a cell with a virus.

4. The peptide of claim 2 or 3 comprising at least 31 amino acids.

5. The peptide of any of claims 1 to 3 with at least one modification selected from acylation, acetylation, linkage to a non-peptidic macromolecular carrier group, amidation, glycosylation; linkage to an adaptor protein, which promotes uptake of the peptide into cells, oxidation, linkage to a hydrophobic group, sulphatation and/or phosphorylation; wherein the modified peptide promotes infection of a cell with a virus.

6. A peptide which is a retro, inverso or retro-inverso peptide, a variant which comprises at least on D-amino acid, a peptide mimetic or a peptide obtainable by multiple synthesis of a peptide of any of claims 1 to 3, which promotes infection of a cell with a virus.

7. A nucleic acid coding for a peptide of any of claims 1 to 4.

8. An expression vector for expression of a peptide of any of claims 1 to 4.

9. An isolated cell comprising an expression vector of claim 7.

10. An antibody against a peptide of any of claims 1 to 6.

11. A medicament comprising a peptide of any of claims 1 to 6, an antibody of claim 10, a nucleic acid of claim 7 or an expression vector of claim 8.

12. The medicament of claim 11 further comprising a vaccine or a vector for gene therapy.

13. The medicament of claim 11 or 12 in the form of an injection, drops, a spray, tablets, suppositories, a cream, an ointment or a gel.

14. A diagnostic comprising a peptide of any of claims 1 to 6, an antibody of claim 10, a nucleic acid of claim 7 or an expression vector of claim 8.

15. The peptide of any of claims 1 to 6 covalently attached to a carrier or support.

16. Use of a peptide of any of claims 1 to 6, an antibody of claim 10, a nucleic acid of claim 7 or an expression vector of claim 8 for the treatment or diagnosis of a viral infection, prostatic dysfunction, carcinoma or the susceptibility of a patient to a viral infection.

17. The use of claim 16, wherein the viral infection is one of a retrovirus.

18. The use of claim 16 or 17, wherein the viral infection is one by HIV (Human Immunodeficiency Virus), Simian Immunodeficiency Viruses (SIV), Murine Leukemia Virus (MuLV), Foamy Virus, Hepatitis B or C Virus (HBV or HCV), Vesicular Stomatitis Virus (VSV), MuLV or Ebola.

19. Use of a peptide of any of claim 1 to 6 for determining the probability of an individual to transmit a virus, especially HIV, during sexual intercourse.

20. Use of a peptide of any of claim 1 to 6 for promoting the entry of a virus, a viral particle, a vector designed for gene therapy and/or a virus-based vaccine into cells *in vitro* or *in vivo.*

21. Use of a peptide of any of claim 1 to 6 for isolating viruses or viral particles.

22. A method for infecting cells with a virus, a viral particle or a vector designed for gene therapy into cells *in vitro* or *in vivo,* comprising incubating the cells with the virus, viral particle or vector in the presence of a peptide of any of claims 1 to 6.

23. A method for isolating viruses or viral particles comprising attaching peptides of any of claims 1 to 6 to a carrier or a support, contacting the carrier or support with a solution under conditions that the virus or viral particles bind to the attached peptides, separating the carrier or support from the solution, optionally washing and eluting bound viruses and viral particles, and optionally determining the number of bound viruses and viral particles.

24. A method for synthesis of a peptide of any of claims 1 to 6 comprising preparation of a library of peptides from sperm and fractionation of the library.
